# EUROPEAN PATENT APPLICATION

(11) **EP 4 342 428 A2**
(43) Date of publication of application: **27.03.2024**
(21) Application number: 23197696.0
(22) Date of filing: 15.09.2023
(51) Int. Cl.: A61F 2/24

(54) **PROSTHETIC HEART VALVE TISSUE DURABILITY STRUCTURE AND METHOD**

(30) Priority: 26.09.2022 US 202263410103 P; 27.08.2023 US 202318456515
(71) Applicant: Medtronic Vascular Inc., Santa Rosa, CA 95403 (US)
(72) Inventor: Olney, Karl L., Santa Ana, 92705 (US); Nigade, Anish S., Santa Rosa, 95403 (US)
(74) Representative: Zimmermann & Partner Patentanwälte mbB

(57) **Abstract**

The techniques of this disclosure generally relate to a valve prosthesis having a stent structure and a prosthetic valve coupled to the stent structure. The stent structure includes an inflow portion having an outflow crown ring and an outflow portion comprising an outflow portion crown ring. The outflow crown ring includes outflow struts and outflow crowns coupled to the outflow struts. The outflow portion crown ring includes outflow portion struts and superior crowns coupled to the outflow portion struts. The valve prosthesis further comprises an inferior tissue bumper covering the outflow crowns and a superior tissue bumper covering the superior crowns. The tissue bumpers provide a buffer, sometimes called a padding or cushion, between the prosthetic valve and outflow crowns and superior crowns. In this manner, the tissue bumpers protect the prosthetic valve from being damaged by the outflow crowns and the superior crowns.

## Description

### CROSS-REFERENCE TO RELATED APPLICATIONS

This application claims the benefit of the filing date of U.S. Provisional Application No. 63/410,103, filed September 26, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

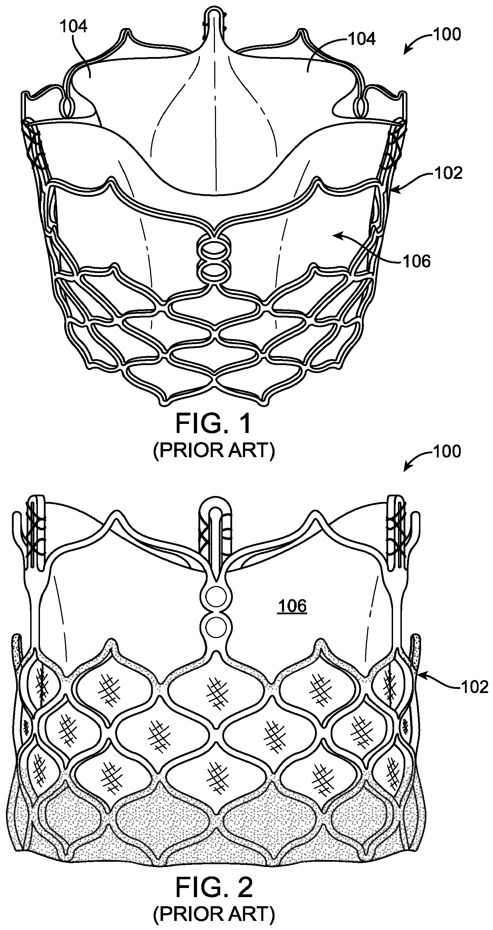

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. For example, when attaching the valve to the frame during valve-frame integration, the valve itself needs to be reinforced to the frame at certain locations without hindering mechanical performance of the frame. Embodiments hereof relate to an improved balloon-expandable transcatheter valve prosthesis configured to minimize tradeoffs between the above-described competing needs.

### SUMMARY

The techniques of this disclosure generally relate to a valve prosthesis having a stent structure and a prosthetic valve coupled to the stent structure. The stent structure includes an inflow portion having an outflow crown ring. The outflow crown ring includes outflow struts and outflow crowns coupled to the outflow struts. The valve prosthesis further comprises an inferior tissue bumper covering the outflow crowns. The inferior tissue bumper provides a buffer, sometimes called a padding or cushion, between the prosthetic valve and the outflow crowns. In this manner, the inferior tissue bumper protects the prosthetic valve from being damaged by the outflow crowns.

In one aspect, the present disclosure provides a valve prosthesis having a stent structure and a prosthetic valve coupled to the stent structure. The stent structure includes an outflow portion having an outflow portion crown ring. The outflow portion crown ring includes outflow portion struts and superior crowns coupled to the outflow portion struts. The valve prosthesis further comprises a superior tissue bumper covering the superior crowns. The superior tissue bumper provides a buffer, sometimes called a padding or cushion, between the prosthetic valve and the superior crowns. In this manner, the superior tissue bumper protects the prosthetic valve from being damaged by the superior crowns.

In one aspect, the present disclosure provides a valve prosthesis comprising a prosthetic valve. The prosthetic valve includes valve leaflets, a valve inflow cylinder proximal of the valve leaflets, and a first support stitching extending in a circumferential direction around the valve inflow cylinder. The first support stitching is less elastic than the valve inflow cylinder. Due to this inelasticity, the first support stitching does not lose tension over time thus preventing creeping and the associated damage of the prosthetic valve.

In another aspect, the present disclosure provides a valve prosthesis including an integral prosthetic valve. The prosthetic valve includes a cylindrical inflow end, valve leaflets, a valve inflow cylinder extending distally from the cylindrical inflow end to the valve leaflets, and an inner skirt extending distally from the cylindrical inflow end. By forming the inner skirt as an integral part of the prosthetic valve, fabrication and attachment of a separate inner skirt is avoided.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a perspective view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve of the valve prosthesis of FIG. 3 in accordance with one embodiment.
FIG. 6 is a side view of the valve prosthesis of FIG. 3 in accordance with one embodiment.
FIG. 7 is a perspective view of a prosthetic valve in accordance with another embodiment.
FIG. 8 is a perspective view of a prosthetic valve in accordance with another embodiment.
FIG. 9 is a laid flat plan view of a tissue coupon illustrating fabrication of the prosthetic valve of FIG. 8 in accordance with one embodiment.
FIG. 10 illustrates a cross-sectional view of a portion of the prosthetic valve of FIG. 8 mounted to a stent structure of FIGS. 3, 6 in accordance with one embodiment.
FIG. 11 is an enlarged view of a region XI of the prosthetic valve of FIG. 8 and an inflow portion cell of the stent structure of FIGS. 3, 6 in accordance with another embodiment.
FIG. 12 is a perspective view of a prosthetic valve in accordance with another embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow.

Although the description is in the context of treatment of an aortic heart valve, embodiments may also be used where it is deemed useful in other valved intraluminal sites that are not in the heart. For example, embodiments may be applied to other heart valves or venous valves as well.

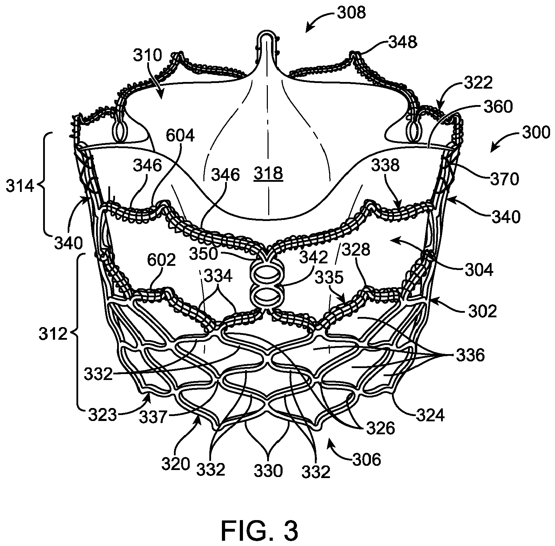

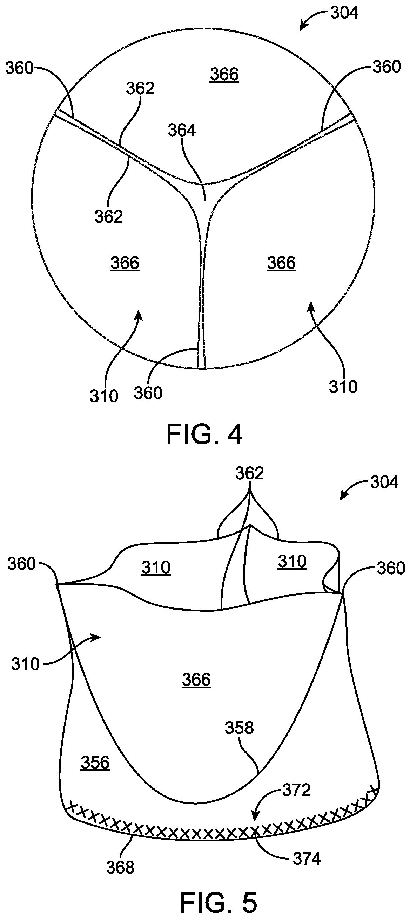

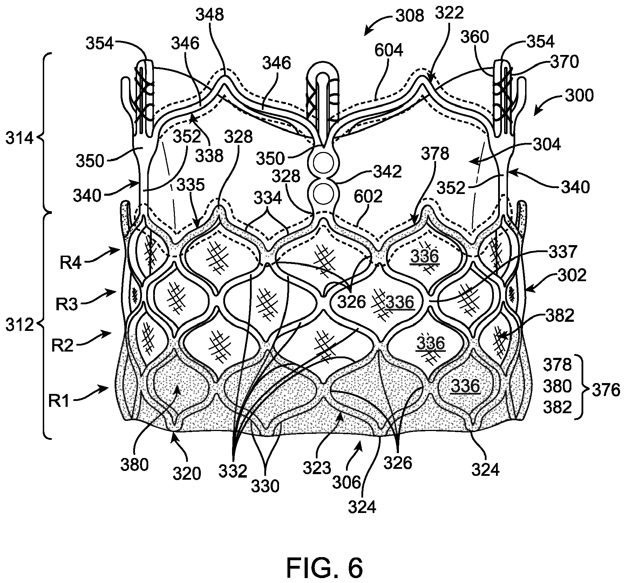

FIG. 3 is a perspective view of a transcatheter valve prosthesis 300 in an expanded configuration in accordance with one embodiment. FIG. 4 is an outflow end view of a prosthetic valve 304 of valve prosthesis 300 of FIG. 3 in accordance with one embodiment. FIG. 5 is a perspective view of prosthetic valve 304 of valve prosthesis 300 of FIG. 3 in accordance with one embodiment. FIG. 6 is a side view of valve prosthesis 300 of FIG. 3 in accordance with one embodiment. FIG. 4 illustrates prosthetic valve 304 in a closed state while FIGS. 3, 5 and 6 illustrates prosthetic valve 304 in an open state.

Referring now to FIGS. 3-6 together, valve prosthesis 300 has a radially-expandable stent structure 302, sometimes called the frame or frame structure, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. Although prosthetic valve 304 is illustrated and discussed herein as having three valve leaflets 310, i.e., prosthetic valve 304 has a tricuspid leaflet configuration, a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway 318, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion 312 further includes an outflow crown ring 335 defined by outflow crowns 328, outflow struts 334, and central crowns 326. Inflow portion 312 generally extends between inflow crown ring 323 and outflow crown ring 335.

In between inflow crown ring 323 and outflow crown ring 335, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, e.g., are sometimes called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 302 and between outflow end 322 and inflow portion 312. Outflow portion 314 includes an outflow portion crown ring 338, commissure posts 340, and non-commissure posts 342. Outflow portion 314 can be configured in a shape that forms a central lumen or passageway.

Outflow portion crown ring 338 includes outflow portion struts 346, superior crowns 348, and inferior crowns 350. Each outflow portion struts 346 is connected at one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow portion crown ring 338 and outflow crowns 328 of inflow portion 312. In accordance with this embodiment, non-commissure posts 342 are shaped as a figure eight and can be used as markers for depth of implant as well as clocking of valve prosthesis 300. As used herein, longitudinally is in a direction parallel with the longitudinal axis, radially is perpendicular and in a radial direction from the longitudinal axis, and circumferentially is in a plane perpendicular to the longitudinal axis and in a direction along the circumference of valve prosthesis 300.

Commissure posts 340 extend longitudinally and include axial frame members 352 and trident posts 354. Axial frame members 352 extend longitudinally between and connect inferior crowns 350 of outflow portion crown ring 338 and outflow crowns 328 of inflow portion 312. Trident posts 354 extend in a cantilever fashion and in the outflow or distal direction from inferior crowns 350 of outflow portion crown ring 338. Axial frame members 352 and trident posts 354 are parallel with one another and are segments of commissure posts 340, which are linear members.

Prosthetic valve 304 is a one piece 3D molded structure in accordance with this embodiment. Illustratively, a single cylindrical piece is molded to form prosthetic valve 304. Accordingly, although various structures of prosthetic valve 304 are discussed below, prosthetic valve 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining un-molded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356.

Prosthetic valve 304 is disposed within and secured to at least trident posts 354 of commissure posts 340 of stent structure 302. In addition, prosthetic valve 304 may also be disposed within and secured to inflow portion 312 of stent structure 302. More particularly, commissures 360 are attached to trident posts 354, e.g., with commissure stitching 370. Further, a margin of attachment (MOA) 372 of valve inflow cylinder 356, e.g., a region directly adjacent cylindrical inflow end 368, is attached to inflow portion 312, e.g., with MOA stitching 374.

Valve prosthesis 300 further includes a skirt 376, e.g., formed of graft material, which encloses or lines a portion of stent structure 302. Skirt 376 is not illustrated in FIG. 3 for clarity of visualization of stent structure 302. Margin of attachment 372 of valve inflow cylinder 356 is sutured or otherwise securely and sealingly attached to the interior surface of skirt 376 and to inflow portion 312, e.g., with MOA stitching 374.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE, which creates a one-way fluid passage. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, and inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, outflow end outer skirt 378 covers first row R1 of inflow portion cells 336. Inner skirt 382 is located on the inner surface of inflow portion 312 and extends between inflow crown ring 323 and outflow crown ring 335.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

Paying particular attention now to FIGS. 3 and 6, valve prosthesis 300 further includes an inferior tissue bumper 602 and a superior tissue bumper 604. In FIG. 6, inferior tissue bumper 602 and superior tissue bumper 604 are indicated in dashed lines to allow visualization of stent structure 302.

In one embodiment, inferior tissue bumper 602 covers outflow crown ring 335 of inflow portion 312. Inferior tissue bumper 602 is one or more tissue fibers that are wrapped around outflow crown ring 335 as illustrated. More particularly, inferior tissue bumper 602 covers outflow crowns 328, outflow struts 334, and central crowns 326.

In one embodiment, superior tissue bumper 604 covers outflow portion crown ring 338 of outflow portion 314. Superior tissue bumper 604 is one or more tissue fibers that are wrapped around outflow portion crown ring 338 as illustrated. More particularly, superior tissue bumper 604 covers outflow portion struts 346, superior crowns 348, and inferior crowns 350.

In one embodiment, tissue bumpers 602, 604 are formed of pericardial material; however, may instead be made of another material. Natural tissue for tissue bumpers 602, 604 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as tissue bumpers 602, 604 include DACRON polyester commercially, other cloth materials, nylon blends, polymeric materials, and vacuum deposition nitinol fabricated materials. One polymeric material from which tissue bumpers 602, 604 can be made is an ultra-high molecular weight polyethylene material. In one embodiment, tissue bumpers 602, 604 are formed of the same or similar material as prosthetic valve 304.

Tissue bumpers 602, 604 provide a buffer, sometimes called a padding or cushion, between prosthetic valve 304 and crown rings 335, 338, respectively. More particularly, tissue bumpers 602, 604 prevent direct contact between prosthetic valve 304 and crown rings 335, 338, respectively. In this manner, tissue bumpers 602, 604 protect prosthetic valve 304 from being damaged by crown rings 335, 338, respectively.

For example, absent tissue bumpers 602, 604, during crimping of valve prosthesis 300 for loading into the delivery system, prosthetic valve 304 may be forced into or otherwise inadvertently contact crown rings 335, 338. This inadvertent contact, and the associated damage to prosthetic valve 304, is avoided by placing tissue bumpers 602, 604 between prosthetic valve 304 and crown rings 335, 338.

In another example, during opening and closing of valve leaflets 310, absent tissue bumpers 602, 604, valve leaflets 310 may contact and be damaged by crown rings 335, 338. This contact, and the associated damage to valve leaflets 310, is avoided by placing tissue bumpers 602, 604 between valve leaflets 310 and crown rings 335, 338.

Although both inferior tissue bumper 602 and superior tissue bumper 604 are illustrated and discussed, in another embodiment, only inferior tissue bumper 602 or superior tissue bumper 604 is provided, and not both.

In yet another embodiment, instead of covering the entire outflow crown ring 335 as described and illustrated, inferior tissue bumper 602 covers only a portion of outflow crown ring 335, e.g., only covers outflow crowns 328. For example, the pointy shape of outflow crowns 328 can cause abrasion and damage to prosthetic valve 304, and so outflow crowns 328 are covered by inferior tissue bumper 602.

In yet another embodiment, instead of covering the entire outflow portion crown ring 338 as described and illustrated, superior tissue bumper 604 covers only a portion of outflow portion crown ring 338, e.g., only covers superior crowns 348. For example, the pointy shape of superior crowns 348 can cause abrasion and damage to prosthetic valve 304, and so superior crowns 348 are covered by superior tissue bumper 604.

In yet another embodiment, superior tissue bumper 604 further covers non-commissure posts 342. By covering non-commissure posts 342 with superior tissue bumper 604, contact between valve leaflets 310 and non-commissure posts 342, and the associated damage to valve leaflets 310, is avoided.

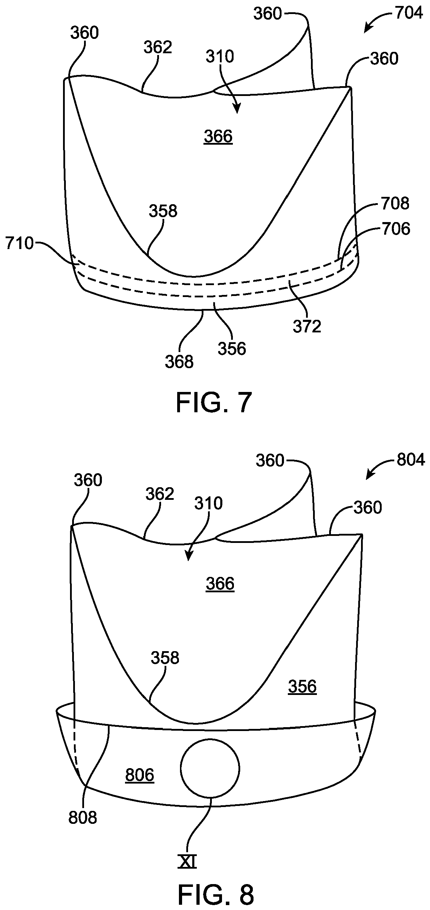

FIG. 7 is a perspective view of a prosthetic valve 704 in accordance with another embodiment. Prosthetic valve 704 of FIG. 7 is similar to prosthetic valve 304 and is coupled to stent structure 302 to form a valve prosthesis similar to valve prosthesis 300 of FIGS. 3-6 as discussed above and only the significant differences are discussed below.

In accordance with this embodiment, prosthetic valve 704 includes support stitchings 706, 708, sometimes called first and second support stitchings 706, 708. Support stitchings 706, 708 extend circumferentially around the entirety of valve inflow cylinder 356. Support stitchings 706, 708 extend in and out of valve inflow cylinder 356 in an alternating repeating pattern, i.e., from the outer surface to the inner surface of valve inflow cylinder 356 through valve inflow cylinder 356. Support stitchings 706,708, e.g., sutures, are inelastic and maintain tension over time.

Absent support stitchings 706, 708, the tissue of prosthetic valve 704 may start to lose its tension and creep over time. In such a case, the tissue would start interacting with stent structure 302 (stent structure 302 is illustrated in FIGS. 3, 6) and be damaged due to this interaction.

Support stitchings 706, 708 are inelastic, e.g., are much less elastic than the material of prosthetic valve 704. Due to this inelasticity, support stitchings 706, 708 will not lose tension over time and thus prevent creeping of prosthetic valve 704. Accordingly, support stitchings 706, 708 prevent damaged due to this creeping of prosthetic valve 704. Generally, support stitchings 706, 708 provide support to valve inflow cylinder 356 of prosthetic valve 704.

Support stitching 706 is longitudinally spaced apart from support stitching 708. In this particular embodiment, support stitching 706 is located proximally from support stitching 708. In one embodiment, a circumferential space 710 between support stitchings 706, 708 forms margin of attachment 372, which is strengthened by support stitchings 706, 708.

Although both support stitching 706 and support stitching 708 are illustrated and discussed, in another embodiment, only support stitching 706 or support stitching 708 is provided. In yet another embodiment, more than two support stitchings similar to support stitchings 706, 708 are provided.

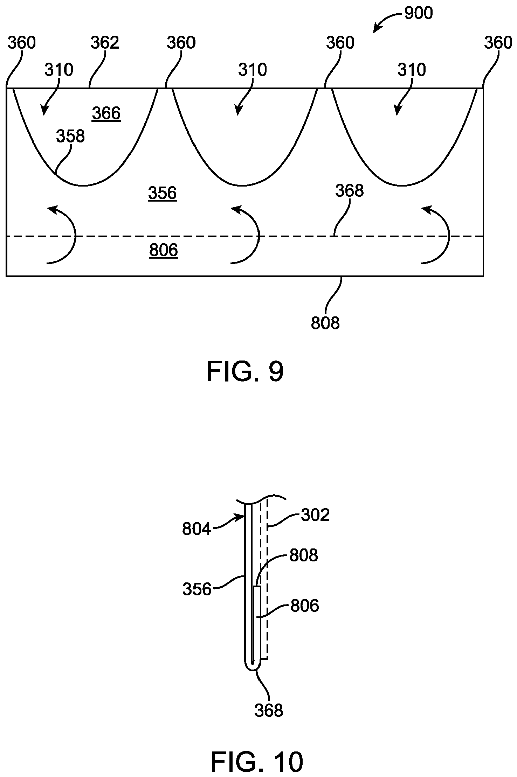

FIG. 8 is a perspective view of a prosthetic valve 804 in accordance with another embodiment. FIG. 9 is a laid flat plan view of a tissue coupon 900 illustrating fabrication of prosthetic valve 804 of FIG. 8 in accordance with one embodiment. Tissue coupon 900 of FIG. 9 corresponds to prosthetic valve 804 of FIG. 8 cut longitudinally at a commissure 360 and prior to folding of an integral inner skirt 806 of prosthetic valve 804. It is to be understood that tissue coupon 900 is set forth in FIG. 9 to facilitate understanding of the fabrication of prosthetic valve 804 of FIG. 8 but that prosthetic valve 804 is fabricated from a cylindrical piece of material and tissue coupon 900 would not exist during actual fabrication. Prosthetic valve 804 of FIG. 8 is similar to prosthetic valve 304 and is coupled to stent structure 302 to form a valve prosthesis similar to valve prosthesis 300 as discussed above in reference to FIGS. 3-6 and only the significant differences are discussed below.

In accordance with this embodiment, prosthetic valve 804 includes integral inner skirt 806 extending distally from cylindrical inflow end 368 and along the outer surface of valve inflow cylinder 356. As illustrated in FIG. 9, to form prosthetic valve 804, prosthetic valve 804 includes an additional length of the material, i.e., inner skirt 806, that is folded over at cylindrical inflow end 368, as indicated in the dashed line in FIG. 9. Inner skirt 806 is cylindrical, e.g., a cylindrical solid strip, extending distally from cylindrical inflow end 368 to a cylindrical outflow end 808 of inner skirt 806.

FIG. 10 illustrates a cross-sectional view of a portion of prosthetic valve 804 of FIG. 8 mounted to stent structure 302 of FIGS. 3, 6 in accordance with one embodiment. As inner skirt 806 is formed from a piece of material extended from valve inflow cylinder 356, inner skirt 806 and valve inflow cylinder 356 have essentially the same diameter and are in abutting contact with one another as illustrated.

As further illustrated in FIG. 10, inner skirt 806 is located between valve inflow cylinder 356 and stent structure 302. In accordance with this embodiment, by forming inner skirt 806 as an integral part of prosthetic valve 804, fabrication and attachment of a separate inner skirt such as inner skirt 382 as illustrated in FIG. 6 is avoided. In this manner, fabrication is simplified.

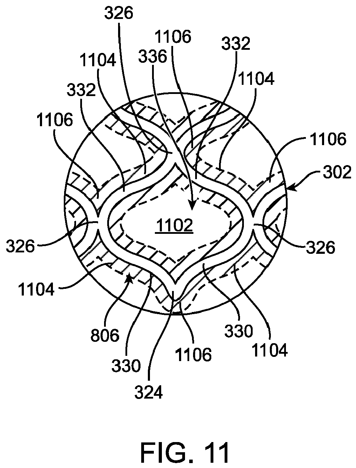

FIG. 11 is an enlarged view of a region XI of prosthetic valve 804 of FIG. 8 and an inflow portion cell 336 of stent structure 302 of FIGS. 3, 6 in accordance with another embodiment. In accordance with this embodiment, instead of being a cylindrical solid strip as illustrated in FIG. 8, inner skirt 806 is cut to have a pattern of inner skirt cells 1102 matching the pattern of inflow portion cells 336. Accordingly, inner skirt 806 includes inner skirt struts 1104 and inner skirt crowns 1106 defining inner skirt cells 1102 corresponding to struts 330, 332, and crowns 324, 326 that define inflow portion cells 336, respectively.

Although only a single inner skirt cell 1102 and a corresponding single inflow portion cell 336 is illustrated in FIG. 11 for simplicity, it is to be understood that the pattern continues for all of the inflow portion cells 336. In one embodiment, only a single row of inner skirt cells 1102 corresponding to first row R1 of inflow portions cells 336 are formed. However, in other embodiments, more than one row of inner skirt cells 1102 are formed, e.g., corresponding to rows R1, R2, R3, and/or row R4 of inflow portion cells 336.

By removing material of inner skirt 806 to form inner skirt cells 1102, the amount of material of inner skirt 806 is reduced thus the profile of inner skirt 806, and more generally of prosthetic valve 804, is improved.

Referring again to FIG. 8, cylindrical outflow end 808 of inner skirt 806 is proximal of cusps 358. In one embodiment, prosthetic valve 804 is a supra-annular valve and thus cusps 358 are higher on stent structure 302 providing space for inner skirt 806 to not interfere with valve leaflets 310. Accordingly, inner skirt 806 overlaps only valve inflow cylinder 356.

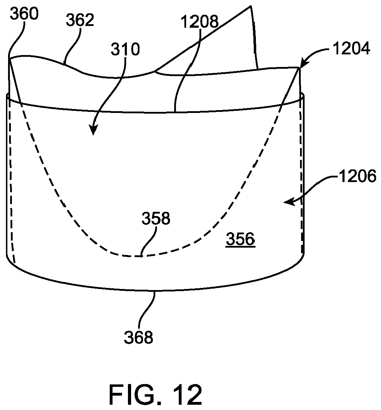

FIG. 12 is a perspective view of a prosthetic valve 1204 in accordance with another embodiment. Prosthetic valve 1204 of FIG. 12 is similar to prosthetic valve 804 of FIG. 8 and only the significant differences are discussed below. More particularly, the length of an inner skirt 1206 of prosthetic valve 1204 of FIG. 12 is greater than the length of inner skirt 806 of prosthetic valve 804 of FIG. 8.

Referring now to FIG. 12, in accordance with this embodiment, a cylindrical outflow end 1208 of inner skirt 1206 is located adjacent to the outflow end of prosthetic valve 1204, e.g., free edges 362 and commissures 360. In other words, inner skirt 1206 overlaps the entirety of the prosthetic valve 1204 including valve inflow cylinder 356, cusps 358, and valve leaflets 310. In other embodiments, cylindrical outflow end 1208 of inner skirt 1206 extends above cusps 358 to overlap valve leaflets 310 but is below free edges 362 and commissures 360.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Aspects and embodiments of the invention may be defined by the following clauses.
Clause 1. A valve prosthesis comprising:
   a stent structure;
   a prosthetic valve coupled to the stent structure, wherein the stent structure comprises:
      an inflow portion comprising an outflow crown ring, the outflow crown ring comprising:
      outflow struts; and
      outflow crowns coupled to the outflow struts; and
   an inferior tissue bumper covering the outflow crowns.
Clause 2. The valve prosthesis of Clause 1 wherein the inferior tissue bumper further covers the outflow struts.
Clause 3. The valve prosthesis of Clause 2 wherein the outflow crown ring further comprises central crowns, the inferior tissue bumper covering the central crowns.
Clause 4. The valve prosthesis of Clause 1 wherein the inferior tissue bumper prevents the prosthetic valve from contacting the outflow crowns.
Clause 5. A valve prosthesis comprising:
   a stent structure;
   a prosthetic valve coupled to the stent structure,
   wherein the stent structure comprises:
      an outflow portion comprising an outflow portion crown ring, the outflow portion crown ring comprising:
      outflow portion struts; and
      superior crowns coupled to the outflow portion struts; and
   a superior tissue bumper covering the superior crowns.
Clause 6. The valve prosthesis of Clause 5 wherein the superior tissue bumper further covers the outflow portion struts.
Clause 7. The valve prosthesis of Clause 5 wherein the outflow portion further comprises non-commissure posts, the superior tissue bumper further covering the non-commissure posts.
Clause 8. A valve prosthesis comprising:
   a prosthetic valve comprising:
   valve leaflets;
   a valve inflow cylinder proximal of the valve leaflets; and
   a first support stitching extending in a circumferential direction around the valve inflow cylinder, the first support stitching being less elastic than the valve inflow cylinder.
Clause 9. The valve prosthesis of Clause 8 wherein the first support stitching supports the valve inflow cylinder.
Clause 10. The valve prosthesis of Clause 8 wherein the first support stitching extends in and out of the valve inflow cylinder from an outer surface of the valve inflow cylinder to an inner surface of the valve inflow cylinder through the valve inflow cylinder.
Clause 11. The valve prosthesis of Clause 8 further comprising a second support stitching extending in the circumferential direction around the valve inflow cylinder, the second support stitching being less elastic than the valve inflow cylinder.
Clause 12. The valve prosthesis of Clause 11 wherein the first support stitching and the second support stitching comprise sutures.
Clause 13. The valve prosthesis of Clause 11 wherein the first support stitching is longitudinally space from the second support stitching.
Clause 14. The valve prosthesis of Clause 13 wherein a circumferential space is defined between the first support stitching and the second support stitching.
Clause 15. The valve prosthesis of Clause 14 further comprising:
   a stent structure; and
   margin of attachment (MOA) stitching coupling the circumferential space to the stent structure.
Clause 16. A valve prosthesis comprising:
   an integral prosthetic valve comprising:
   a cylindrical inflow end;
   valve leaflets;
   a valve inflow cylinder extending distally from the cylindrical inflow end to the valve leaflets; and
   an inner skirt extending distally from the cylindrical inflow end.
Clause 17. The valve prosthesis of Clause 16 further comprising a stent structure coupled to the integral prosthetic valve, the inner skirt being between the valve inflow cylinder and the stent structure.
Clause 18. The valve prosthesis of Clause 17 wherein the stent structure comprises a row of inflow portion cells, the inner skirt comprising inner skirt cells matching the inflow portion cells.
Clause 19. The valve prosthesis of Clause 16 further comprising cusps between the valve leaflets and the valve inflow cylinder, the inner skirt extending distally to a cylindrical outflow end, the cylindrical outflow end being proximal of the cusps.
Clause 20. The valve prosthesis of Clause 16 wherein the inner skirt overlaps the valve leaflets.

Further disclosed herein is a valve prosthesis having a stent structure and a prosthetic valve coupled to the stent structure. The stent structure includes an inflow portion having an outflow crown ring and an outflow portion comprising an outflow portion crown ring. The outflow crown ring includes outflow struts and outflow crowns coupled to the outflow struts. The outflow portion crown ring includes outflow portion struts and superior crowns coupled to the outflow portion struts. The valve prosthesis further comprises an inferior tissue bumper covering the outflow crowns and a superior tissue bumper covering the superior crowns. The tissue bumpers provide a buffer, sometimes called a padding or cushion, between the prosthetic valve and outflow crowns and superior crowns. In this manner, the tissue bumpers protect the prosthetic valve from being damaged by the outflow crowns and the superior crowns.

## Claims

1. A valve prosthesis comprising:
a stent structure;
a prosthetic valve coupled to the stent structure, wherein the stent structure comprises:
an inflow portion comprising an outflow crown ring, the outflow crown ring comprising:
outflow struts; and
outflow crowns coupled to the outflow struts; and
an inferior tissue bumper covering the outflow crowns.

2. The valve prosthesis of Claim 1 wherein the inferior tissue bumper further covers the outflow struts; and/or wherein the outflow crown ring further comprises central crowns, the inferior tissue bumper covering the central crowns.

3. The valve prosthesis of Claim 1 or 2 wherein the inferior tissue bumper prevents the prosthetic valve from contacting the outflow crowns.

4. A valve prosthesis comprising:
a stent structure;
a prosthetic valve coupled to the stent structure,
wherein the stent structure comprises:
an outflow portion comprising an outflow portion crown ring, the outflow portion crown ring comprising:
outflow portion struts; and
superior crowns coupled to the outflow portion struts; and
a superior tissue bumper covering the superior crowns.

5. The valve prosthesis of Claim 4 wherein the superior tissue bumper further covers the outflow portion struts.

6. The valve prosthesis of Claim 4 or 5 wherein the outflow portion further comprises non-commissure posts, the superior tissue bumper further covering the non-commissure posts.

7. A valve prosthesis comprising:
a prosthetic valve comprising:
valve leaflets;
a valve inflow cylinder proximal of the valve leaflets; and
a first support stitching extending in a circumferential direction around the valve inflow cylinder, the first support stitching being less elastic than the valve inflow cylinder.

8. The valve prosthesis of Claim 7 wherein the first support stitching supports the valve inflow cylinder; and/or wherein the first support stitching extends in and out of the valve inflow cylinder from an outer surface of the valve inflow cylinder to an inner surface of the valve inflow cylinder through the valve inflow cylinder.

9. The valve prosthesis of Claim 7 or 8 further comprising a second support stitching extending in the circumferential direction around the valve inflow cylinder, the second support stitching being less elastic than the valve inflow cylinder; and optionally wherein the first support stitching and the second support stitching comprise sutures.

10. The valve prosthesis of Claim 9 wherein the first support stitching is longitudinally spaced from the second support stitching.

11. The valve prosthesis of Claim 9 or 10 wherein a circumferential space is defined between the first support stitching and the second support stitching; and optionally further comprising:
a stent structure; and
margin of attachment (MOA) stitching coupling the circumferential space to the stent structure.

12. A valve prosthesis comprising:
an integral prosthetic valve comprising:
a cylindrical inflow end;
valve leaflets;
a valve inflow cylinder extending distally from the cylindrical inflow end to the valve leaflets; and
an inner skirt extending distally from the cylindrical inflow end.

13. The valve prosthesis of Claim 12 further comprising a stent structure coupled to the integral prosthetic valve, the inner skirt being between the valve inflow cylinder and the stent structure.

14. The valve prosthesis of Claim 13 wherein the stent structure comprises a row of inflow portion cells, the inner skirt comprising inner skirt cells matching the inflow portion cells.

15. The valve prosthesis of any one of Claims 12 to 14 further comprising cusps between the valve leaflets and the valve inflow cylinder, the inner skirt extending distally to a cylindrical outflow end, the cylindrical outflow end being proximal of the cusps; and/or wherein the inner skirt overlaps the valve leaflets.
